(19) Europäisches Patentamt
European Patent Office
Office européen des brevets

(11) **EP 3 301 611 B1**

(12) **EUROPEAN PATENT SPECIFICATION**

(45) Date of publication and mention
of the grant of the patent:
**10.11.2021 Bulletin 2021/45**

(51) Int Cl.:
**G06K 9/00** (2006.01)　　　**G06K 9/62** (2006.01)
**G06N 3/04** (2006.01)

(21) Application number: **17193073.8**

(22) Date of filing: **26.09.2017**

(54) **ARTIFICIAL NEURAL NETWORKS FOR HUMAN ACTIVITY RECOGNITION**

KÜNSTLICHE NEURONALE NETZWERKE ZUR ERKENNUNG MENSCHLICHER AKTIVITÄTEN

RÉSEAUX NEURONAUX ARTIFICIELS POUR LA RECONNAISSANCE D'ACTIVITÉS HUMAINES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.09.2016 US 201615280463**

(43) Date of publication of application:
**04.04.2018 Bulletin 2018/14**

(73) Proprietor: **STMicroelectronics S.r.l.**
**20864 Agrate Brianza (MB) (IT)**

(72) Inventors:
• **PAU, Danilo Pietro**
**20099 Sesto San Giovanni (Milano) (IT)**
• **PLEBANI, Emanuele**
**24039 Sotto il Monte Giovanni XXIII (Bergamo) (IT)**

(74) Representative: **Bosotti, Luciano et al**
**Buzzi, Notaro & Antonielli d'Oulx S.p.A.**
**Corso Vittorio Emanuele II, 6**
**10123 Torino (IT)**

(56) References cited:
• MUHAMMAD SHOAIB ET AL: "Towards Physical Activity Recognition Using Smartphone Sensors", 2013 IEEE 10TH INTERNATIONAL CONFERENCE ON UBIQUITOUS INTELLIGENCE AND COMPUTING AND 2013 IEEE 10TH INTERNATIONAL CONFERENCE ON AUTONOMIC AND TRUSTED COMPUTING, 18 December 2013 (2013-12-18), pages 80-87, XP055446152, DOI: 10.1109/UIC-ATC.2013.43 ISBN: 978-1-4799-2481-3
• FRANCISCO ORD??EZ ET AL: "Deep Convolutional and LSTM Recurrent Neural Networks for Multimodal Wearable Activity Recognition", SENSORS, vol. 16, no. 1, 18 January 2016 (2016-01-18), page 115, XP055368352, DOI: 10.3390/s16010115
• ANDREAS BULLING ET AL: "A tutorial on human activity recognition using body-worn inertial sensors", ACM COMPUTING SURVEYS, ACM, NEW YORK, NY, US, US, vol. 46, no. 3, 1 January 2014 (2014-01-01), pages 1-33, XP058043677, ISSN: 0360-0300, DOI: 10.1145/2499621

EP 3 301 611 B1

**Description**

BACKGROUND

Technical Field

The present disclosure relates to techniques, devices and systems for recognizing human activity based on sensed information, such as signals acquired by wearable sensor devices.

Description of the Related Art

Wearable sensor device may continuously acquire and store information, and periodically transmit signals conveying the information, which may be processed in real time, for example, remotely by a host device or locally by the wearable device itself. Activities in which a wearer is engaged may be detected based on the information conveyed by the sensor signals. The signals may convey physiological data, acceleration data, rotation data, position data, etc.
The information may be analyzed to determine one or more activities in which the wearer is engaged. For example, a wearable sensor device may include one or more accelerometers, and an activity of the wearer may be determined based on accelerometer data from the one or more accelerometers, complemented by one or more gyroscopes. Simplest models may typically be employed to determine an activity of a wearer based on accelerometer data.
A method for the use of a smartphone accelerometer in physical activity recognition is disclosed in Muhammad Shaoib, et al:". Towards Physical Activity Recognition Using Smartphone Sensors", 2013 IEEE 10th International Conference on Ubiquitous Intelligence and Computing, UIC 2013, pages 80-87. The role of a gyroscope and a magnetometer, both when used alone as well as in combination with an accelerometer is discussed. For this purpose, the document investigates the role of these three smartphone sensors in activity recognition. The document evaluates their roles on four body positions using seven classifiers while recognizing six physical activities. The document shows that in general an accelerometer and a gyroscope complement each other, thereby making the recognition process more reliable, while also noting that, in most cases, a gyroscope does not only improve the recognition accuracy in combination with an accelerometer, but it also achieves a reasonable performance when used alone. Based on the disclosed evaluations, the document shows that it is difficult to make an exact general statement about which sensor performs better than the others in all situations because their recognition performance depends on the smartphone's position, the selected classifier, and the activity being recognized.
Francisco Ordóñez et al: "Deep Convolutional and LSTM recurrent Neural Networks for Multimodal Wearable Activity recognition", Sensors, vol. 16, no. 1, page 115 discuss a generic deep framework for activity recognition based on convolutional and LSTM recurrent units, which: (i) is suitable for multimodal wearable sensors; (ii) can perform sensor fusion naturally; (iii) does not require expert knowledge in designing features; and (iv) explicitly models the temporal dynamics of feature activations. The document evaluates the framework on two datasets, one of which has been used in a public activity recognition challenge.
Finally, Andreas Bullig et al: "A tutorial on human activity recognition using body-worn inertial sensor", ACM Computing Surveys, ACM, New York, vol. 46, no. 3, pages 1-33 discuss research activity in the field of human activity recognition. A comprehensive hands-on introduction to the field of human activity recognition is provided by specifically focusing on activity recognition using on-body inertial sensors. The document describes the concept of an Activity Recognition Chain (ARC) as a general-purpose framework for designing and evaluating activity recognition systems.

BRIEF SUMMARY

The present invention has an objective to recognize human activity based on sensed information where each class activity is recognized without bias towards the most frequent classes. This objective is achieved by the subject-matter of the independent claims. The dependent claims contain advantageous aspects of the present invention.

BRIEF DESCRIPTION OF THE SEVERAL VIEWS OF THE DRAWINGS

Figure 1 is a functional block diagram of an embodiment of a system to detect user activities based on information gathered by a wearable device.
Figure 2 illustrates an embodiment of a method of equalizing a number of samples in a data set.
Figure 3 illustrates an embodiment of a classification pipeline to classify samples.
Figure 4 illustrates an embodiment of a neuron structure.

Figure 5 illustrates an embodiment of a structure of fully connected layer architecture.

Figure 6 illustrates an embodiment of a logistic sigmoid function.

Figure 7 illustrates an embodiment of a convolutional layer employing a sliding window.

Figure 8 illustrates an embodiment of the structure of a convolutional network.

Figure 9 illustrates an embodiment of a recurrent neural Echo State Network (ESN) pipeline. This embodiment is not covered by the claims.

Figure 10 illustrates an embodiment of a convolutional neural network (CNN) pipeline.

Figure 11 illustrates a portion of the embodiment of a CNN of Figure 10 in more detail.

Figure 12 illustrates an embodiment of a partial CNN computation process that may be employed by the embodiment of a CNN of Figure 10.

Figure 13 illustrates an embodiment of a logical diagram of a partial computation of a CNN network.

Figure 14 illustrates an example distribution of a computational load of an embodiment.

Figure 15 illustrates an example histogram of a label distribution on a window and a label selected by a median filter of an embodiment of a CNN pipeline.

Figure 16 illustrates an embodiment of an encoding of a Finite State Machine (FSM), or a Finite State Automata (FSA).

Figure 17 illustrates an embodiment of a convolutional neural network CNN pipeline which receives accelerometer and gyroscope data.

Figure 18 illustrates an embodiment of a convolutional neural network CNN pipeline employing a global mean pooling layer.

DETAILED DESCRIPTION

In the following description, certain details are set forth in order to provide a thorough understanding of various embodiments of devices, systems, methods and articles. However, one of skill in the art will understand that other embodiments may be practiced without these details. In other instances, well-known structures and methods associated with, for example, wearable devices and signal processing circuitry, such as transistors, multipliers, transmitters, NFC circuits, integrated circuits, etc., have not been shown or described in detail in some figures to avoid unnecessarily obscuring descriptions of the embodiments.

Unless the context requires otherwise, throughout the specification and claims which follow, the word "comprise" and variations thereof, such as "comprising," and "comprises," are to be construed in an open, inclusive sense, that is, as "including, but not limited to."

Reference throughout this specification to "one embodiment," or "an embodiment" means that a particular feature, structure or characteristic described in connection with the embodiment is included in at least one embodiment. Thus, the appearances of the phrases "in one embodiment," or "in an embodiment" in various places throughout this specification are not necessarily referring to the same embodiment, or to all embodiments. Furthermore, the particular features, structures, or characteristics may be combined in any suitable manner in one or more embodiments to obtain further embodiments.

The headings are provided for convenience only, and do not interpret the scope or meaning of this disclosure.

The sizes and relative positions of elements in the drawings are not necessarily drawn to scale. For example, the shapes of various elements and angles are not drawn to scale, and some of these elements are enlarged and positioned to improve drawing legibility. Further, the particular shapes of the elements as drawn are not necessarily intended to convey any information regarding the actual shape of particular elements, and have been selected solely for ease of recognition in the drawings.

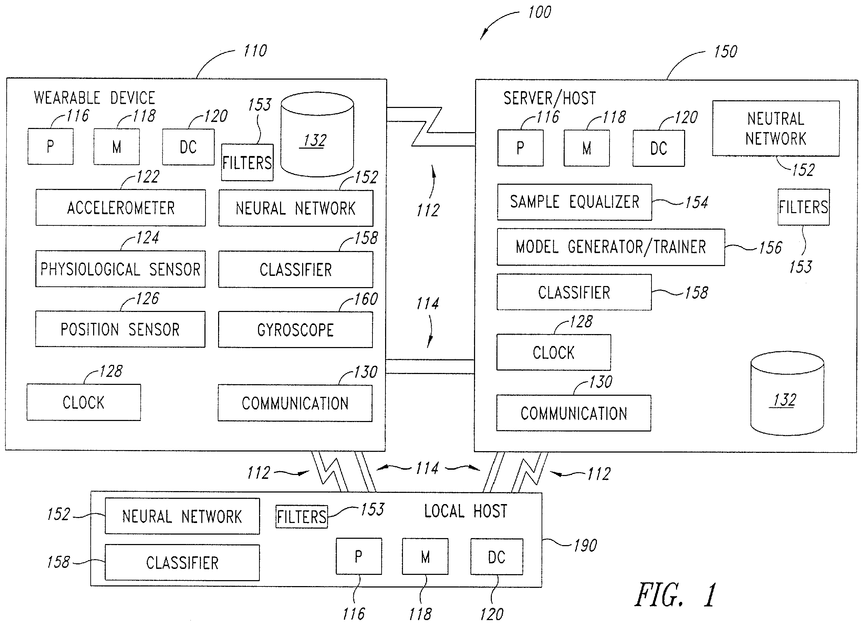

Figure 1 is a functional block diagram of an embodiment of a system 100 to determine an activity of a wearer of a wearable device. As illustrated, the system 100 comprises a wearable sensor device 110, a host/server 150, and an optional local host 190. The wearable sensor device 110, the host/server 150, and the optional local host 190 as illustrated are communicatively coupled together by one or more wireless communication links 112 and one or more wired communication links 114, which may be used alone or in various combinations to facilitate transmission of control and data signals between the wearable device 110, the host/server 150 and the optional local host 190. The optional local host may facilitate communication between the wearable device 110 and the host/server 150, for example, when the host/server 150 is a remote server. As illustrated, the wearable sensor device 110, the host/server 150 and the optional local host include circuitry comprising one or more processors or processor cores P 116, one or more memories M 118, and discrete circuitry DC 120, such as one or more adders, one or more multipliers, one or more filters (such as band pass, low pass, high pass filters, infinite impulse response (IIR), finite impulse (FIR) filters, etc.), etc., which may, alone or in various combinations, implement one or more functions of the system 100.

As illustrated, the wearable device 110 comprises circuitry including one or more accelerometers 122, such as a triaxle accelerometer, one or more gyroscopes, such as the illustrated triaxle gyroscope 160, etc., one or more physiological sensors 124, such as a heart rate sensor, a temperature sensor, a respiration sensor, etc., one or more position sensors 126, such as a global position sensor, etc., one or more clocks 128, one or more communication circuits 130, and one or more database structures 132. For ease of illustration, other circuitry, which may typically be included in a wearable device such as the wearable device 110, are omitted from Figure 1. Such circuitry may include bus systems, power systems, interfaces, etc.

The one or more accelerometers 122 sense movement data and generate signals indicative of the movement data, such as acceleration data for three axis of movement x, y and z of the wearable device 110. Multiple accelerometers 122 may be employed, such as an accelerometer for each axis, an accelerometer configured to gather acceleration data along two axes, together with an accelerometer to gather acceleration data along a third axis, an accelerometer configured to gather acceleration data alone three axes, etc., and various combinations thereof.

The one or more physiological sensors 124 gather physiological data, such as heart rate information, temperature information, respiration information, etc., related to a wearer of the wearable device 110. The one or more position sensors 126 gather position data, such as a data related to a geographical position of the wearable device 110, data related to a position of the wearable device 110 on a wearer, etc. The one or more clocks 128 provide timing signals to facilitate the collection of data samples, for use, for example, in generating training data, determining wearer activities associated with the data samples, time stamps etc. The one or more communication circuits 130 facilitate the exchange of data and control signals between components of the system 100, and may include, for example, near-field communication circuits, serial bus communication circuits, LAN and WAN communication circuits, wi-fi communication circuits, mobile telephone communication circuits, etc., and various combinations thereof. The one or more data structures 132 may store, for example, training data, test data, control parameters, such as modeling parameters, instructions (for example, for execution by the one or more processors P 116), etc., and various combinations thereof.

The one or more gyroscopes 160 sense orientation data and generate signals indicative of the orientation data, such as orientation data with respect to three axis of a three-dimensional space x, y and z of the wearable device 110. Multiple gyroscopes 160 may be employed, such as a gyroscope for each axis.

As illustrated, the server/host 150 comprises circuitry including a neural network 152, one or more filter banks 153, such as IIR filters, FIR filters, etc., a sample equalizer 154, a model generator/trainer 156, one or more data structures 132, a classifier 158, which may classify a data sample based on a model generated by the model generator 156 and/or data stored in the one or more data structures 132 as being associated with one or more activities (e.g., stationary, walking, fast walking, jogging, biking, driving, etc.), one or more clocks 128 and communication circuitry 130. For ease of illustration, other circuitry, which may typically be included in a server/host such as the server/host 150, are omitted from Figure 1. Such circuitry may include bus systems, power systems, interfaces, etc.

In some embodiments, one or more functions of the wearable device 110, of the server/host 150 and/or of the optional local host 190 may instead or also be performed by another of the wearable device 110, the server/host 150 and/or the optional local host 190. For example, in some embodiments, the wearable device and/or the local host may include a neural network, such as the neural network 152, and a classifier, such as the classifier 158, similar to those of the server/host 150.

The inventors have recognized that the number of samples in training data, such as data used to train a neural network to classify data samples, as well as the number of samples in test data sets to be classified (which may also be employed to update models) may tend to be skewed in favor of activities of a particular class. Table 1, below, illustrates the number of samples associated with various activity classes in an example training data set, referred to herein after as Dataset 1.1, Training Set.

Table 1: Example Dataset 1.1, Training Set Distribution

| Activity Class | Number of Samples | Percentage of Total Samples |
| --- | --- | --- |
| Stationary | 4,536,217 | 46.44 |
| Walking | 1,448,165 | 14.83 |
| Jogging | 1,679,551 | 17.20 |
| Biking | 594,737 | 6.09 |
| Driving | 1,508,601 | 15.45 |

Table 2, below, illustrates a number of samples associated with various activity classes for a first test data set, referred to hereinafter as Dataset 1.1, Test Set 1.

Table 2: Example Dataset 1.1, Test Set 1 Distribution

| Activity Class | Number of Samples | Percentage of Total Samples |
|---|---|---|
| Stationary | 216,048 | 40.68 |
| Walking | 81,018 | 15.25 |
| Jogging | 81,018 | 15.25 |
| Biking | 72,016 | 13.56 |
| Driving | 81,018 | 15.25 |

Table 3, below, illustrates a number of samples associated with various activity classes for a second test data set, referred to hereinafter as Dataset 1.1, Test Set 2.

Table 3: Example Data Set 1.1, Test Set 2 Distribution

| Activity Class | Number of Samples | Percentage of Total Samples |
|---|---|---|
| Stationary | 207,046 | 39.66 |
| Walking | 81,018 | 15.52 |
| Jogging | 81,018 | 15.52 |
| Biking | 72,016 | 13.79 |
| Driving | 81,018 | 15.52 |

The data sequences used to generate the training and test data sets were acquired at a frequency of 50 Hz, but other acquisition frequencies may be employed, such as, for example, 16 Hz. For training and test purposes, the data sequences were decimated at a 1:3 ratio, generating sequences having an acquisition frequency of approximately 16 Hz. The training data set in the example of Table 1 was generated from 434 data sequences organized into five classes: stationary, walking, jogging, biking and driving. The test data set in the example of Table 2 was generated from 59 acquired data sequences not included in the training data set, and organized into the same five classes. The test data set in the example of Table 3 was generated from 58 acquired data sequences, which may include sequences included in the training data set, and organized into the same five classes.

Table 4, below, illustrates a number of samples associated with various activities for cleaned-up test data set, and includes a column showing a number of samples in the training data set. The cleaned-up test data set in the example of Table 4 was generated from 1054 acquired data sequences and organized into the same five classes. The distribution of samples is skewed toward the stationary and walking sequences. For ease of reference, the cleaned up test data set of Table 4 will be referred to hereinafter as Dataset 2.0, Cleaned-Up Test Set 1.

Table 4: Dataset 2.0, Cleaned-Up Test Set 1 Distribution

| Activity | Number of Samples | Percentage of Total Samples | Training Data Set Samples |
|---|---|---|---|
| Stationary | 8,674,636 | 80.11 | 4,536,217 |
| Walking | 1,844,416 | 17.03 | 1,448,165 |
| Jogging | 23,350 | 00.22 | 1,679,551 |
| Biking | 49,052 | 00.45 | 594,737 |
| Driving | 237,372 | 02.19 | 1,508,601 |

In the training of a model, the training error is often computed as the sum of the errors of each sample. If, as in the case of both the first test data set, Dataset 1.1, Test Set 1, and the Dataset 2.0, Cleaned-Up Test Set 1, some classes are more represented, the training error promotes those classes to the detriment of the least represented classes. For example, in the case of the Cleaned-Up Test Set of Table 4, a True Positive Rate (TPR) of 80.11% can be reached by predicting "stationary" all the time. To facilitate avoiding this issue, the data set is equalized, that is, the same number of samples (or approximately the same number of samples) may be selected for each class.

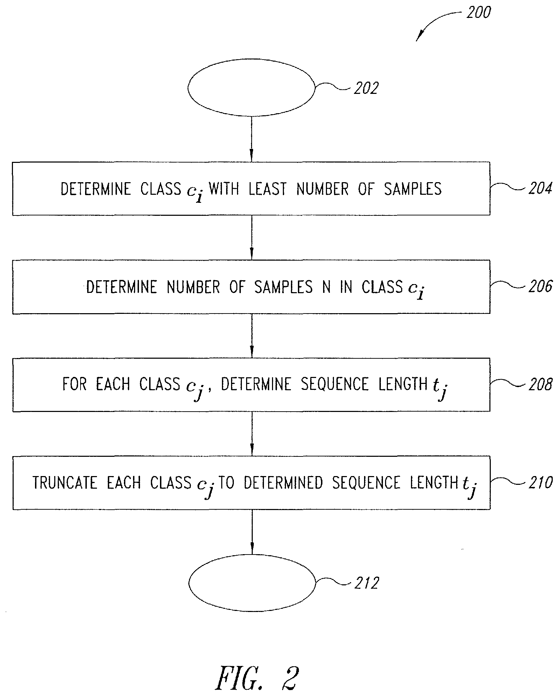

Figure 2 illustrates an embodiment of a method 200, which may be employed by the system 100 of Figure 1 (e.g., by the sample equalizer 154), to equalize the number of samples in a data set, such as a data set used for training or for evaluating the performing of the system 100. For convenience, Figure 2 will be described with reference to the system 100 of Figure 1 and the training data set of Table 1. Other embodiments of systems and devices may employ the method 200 of Figure 2 with other data sets.

The method 200 proceeds from 202 to 204. At 204, the system 100 determines a class $c_i$ having a fewest number of samples N. In the example of the training data set of Table 1, Biking would be selected as the class $c_i$ having the fewest number of samples N.

The method 200 proceeds from 204 to 206. At 206, the system 100 determines the number of samples N in the determined class $c_i$. In the example of the training data set of Table 1, the number of samples N in the Biking class $c_i$ would be determined to be 594,737.

The method 200 proceeds from 206 to 208. At 208, the system 100 determines, for each class $c_j$ other than the determined class $c_i$, a sequence length $t_j$ such that the total number of samples $N_j$ for the class $c_j$, assuming the sequences of the class $c_j$ are truncated at sequence length $t_j$, equals or just exceeds the number N of samples in the class $c_i$. In other words, $N_j < N$ for length $t_j$ - 1. Thus, $t_j$ for each class other than the determined class is determined based on the number of samples N in the determined class.

The method 200 proceeds from 208 to 210. At 210, the system 100, for each class $c_j$ other than the determined class $c_i$, truncates all of the sequences in the class $c_j$ to the determined sequence length $t_j$ for the class $c_j$. In other words, samples in each sequence for each class $c_j$ beyond the determined length $t_j$ are discarded.

The method 200 proceeds from 210 to 212, where the system 100 may perform other processing, such as returning an equalized data set for use in training or evaluating a model, such as a neural network model.

Embodiments of the method 200 may include other acts not shown in Figure 2, and may perform the acts shown in Figure 2 in various orders. For example, in some embodiments acts 204 and 206 may be combined. Equalizing the number of samples by truncating the sequence lengths facilitates discarding whole sequences during the sample selection process.

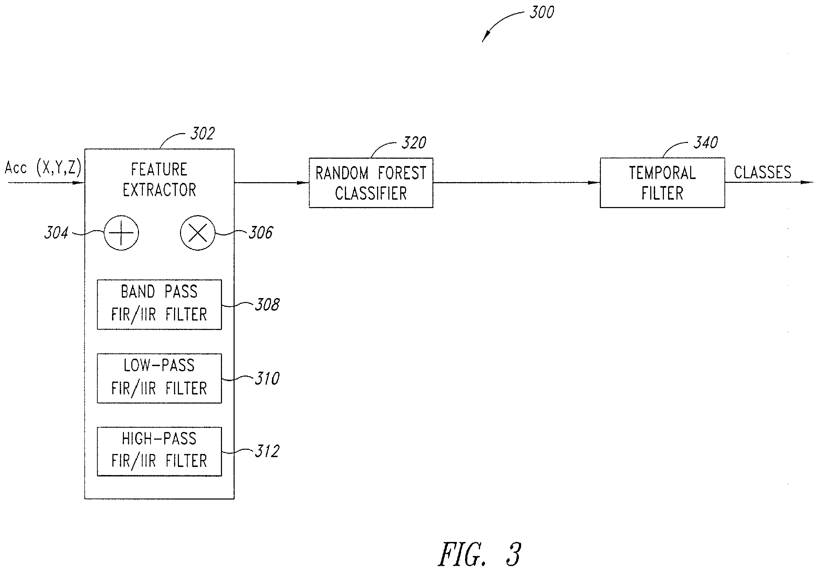

Figure 3 illustrates an embodiment of a classification pipeline circuit 300 which may be employed, for example, by an embodiment of a system, such as the system 100 of Figure 1 (e.g., by the classifier 158), to classify samples. For convenience, the classification pipeline 300 will be described with reference to the system 100 of Figure 1 and with reference to acceleration data. Other embodiments of systems and devices may employ the classification pipeline 300 of Figure 3 with other and/or additional activity-related data, such as acceleration data, orientation data, geographical position data, physiological data, etc., and various combinations thereof.

Acceleration data is received by a feature extractor 302. As illustrated, the feature extractor 302 includes one or more adders 304, one or more multipliers 306, one or more band-pass FIR/IIR filters 308, one or more low-pass FIR/IIR filters 310 and one or more high-pass FIR/IIR filters 312. Other mathematic operation circuits may be employed, such as dividers, square root operators, etc.

The acceleration data may be received by the feature extractor 302 in the form of input vectors a normalized with respect to a gravity acceleration g, with vectors a having a component in each of three axis and expressed in m/s$^2$ (e.g., $a = [a_x, a_y, a_z]$). For convenience, vectors may be indicated with bold typeface herein. In an embodiment, the feature extractor computes an acceleration norm A for each received input as follows:

$$A = \mathbf{a} \cdot \mathbf{a}$$

In an embodiment, the signal A is filtered with a band-pass FIR/IIR filter 308 and/or a low-pass FIR/IIR filter 310 and/or a high-pass FIR/IIR filter 312. As illustrated, the feature extractor provides extracted feature information to a random forest classifier 320 and to a temporal filter 340.

The random forest classifier 320 in an embodiment may comprise a multitude of decision trees, which discriminate classes. In an embodiment, the random forest trees may be implemented using complex sequences hard-coded *if-then-else* structures in C language. In an embodiment, the temporal filter 340 handles special cases based on the output of the random forest classifier 320 and corrects the output of the random forest classifier 320 in case of misclassification errors by filtering the current, future and previous classification samples (in time order) to output the correct class recognized: for example, some data at the output of 320 may indicate a classification output should be stationary, even if previous classification data indicates a different class. The output of the temporal filter may provide a more correct and coherent series of classifications of a current activity by exploiting temporal correlation of various previous decisions at the output of the random forest 320 that otherwise would be incoherent if left as they are. For example the random forest classifier 320 may output 6 (subsequent in time) classifications in the following sequence: *running, running, driving, running, running, running etc.* Clearly the 3rd classification output *(driving)* is highly incoherent with the previous and future ones and therefore will be replaced at the output of the temporal filter 340 with *running* so that the mistake is corrected.

Training of artificial neural network architectures on raw acceleration data was performed in a study in an end-to-end fashion without input sample pre-processing and feature design. Two architectures were evaluated: an Echo State Network (ESN) having a reservoir, three inputs (one for each acceleration axis) and an output trained through logistic regression; and a Convolutional Neural Network having four layers.

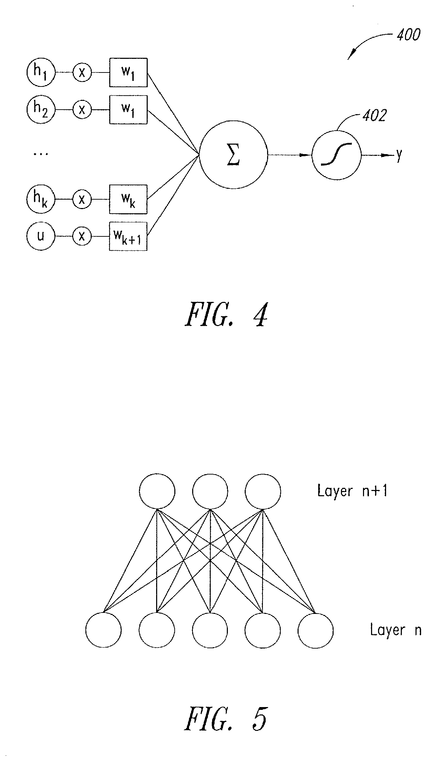

A typical neuron structure 400 of an embodiment is represented in Figure 4. A set of inputs may comprise outputs of other neurons $h_i$, and input signals u, and are weighted by a set of values $w_1$, $w_2$, ... $w_{k+1}$, and then summed. The outputs of other neurons provides as input (the $h_i$ values) may be, for example, generated exclusively by neurons in a previous level or layer in an Acyclic Connected Graph (ACG) structure of a feed forward network, generated from values computed in the same layer in a previous time step in a recurrent network, etc. The summed value is then subjected to a non-linearity function $f(x)$ 402, as illustrated a sigmoid function as a non-limiting example since other functions may be used such as ReLU (Rectified Linear Unit), generating an output of the neuron y.

The outputs $y_j$ of multiple neurons may be generated by stacking weights in a weight matrix $W$, determining a matrix product of the inputs and the weight matrix, generating as vector, and applying the nonlinearity element-wise on the vector. In an embodiment, a bias term b may be added to the neuron to compensate for the mean value of the input.

For a feed-forward network, the output of a neuron layer may be represented as:

$$y_l = f(W \cdot x_{l-1} + b)$$

where $x_{l-1}$ is the output of the previous layer (the input is $x_0$) and $y_l$ is the output of the current layer.

For a recurrent network, the output $y_t$ of a neuron layer may be represented as:

$$y_t = f(W_x \cdot x_t + W_h \cdot y_{t-1} + b)$$

where $x_t$ is the input at the current time step, $y_{t-1}$ is the output of the layer in the previous time step. The neuron input is split between the forward term $W_x$ and the recurrent term $W_h$, which depends on the layer output in the previous time step.

A number of different nonlinearities $f(x)$ may be employed in various embodiments. For example, a Rectified Linear Unit (ReLU) function may be employed. An example ReLU function returns zero for negative values, and does not change the value otherwise, and may be represented mathematically as follows:

$$f(x) = \max(0, x).$$

A sigmoid function, such as a logistic function (S-shaped curve) with a mid-point at zero and a maximum of 1, may be employed. An example sigmoid function may be represented mathematically as follows:

$$f(x) = \frac{1}{1 + e^{-x}}$$

A hyperbolic tangent function may be employed. An example hyperbolic tangent function may be represented mathematically as follows:

$$f(x) = \tanh(x).$$

The *ReLU* nonlinearity may typically be employed in feed-forward networks, and the sigmoid or tanh nonlinearities may typically be employed in recurrent networks.

A Multi-Layer Perceptron (MLP) also known as fully connected architecture may be the simplest neural architecture, where a collection of neurons (layer) compute the output values from a previous (input) layer; the first layer is the input data, and the last layer is the prediction. Multiple layers can be stacked to compute increasing complex functions of the input. Because the output of a layer depends only on the values computed by the previous layer, the architecture is a feed-forward architecture. If a MLP is used as part of a larger network, each layer of the MLP may be referred to as a dense layer or a fully connected layer.

Figure 5 illustrates an example structure of a MLP architecture of an embodiment.

The *sigmoid* nonlinearity takes only values between 0 and 1 and thus can be interpreted as a probability estimate in a binary classification problem, e.g. a problem where only two classes are available. However, for multiple classes a tool which can estimate the probabilities over n classes is needed. In an embodiment, a softmax layer may be employed. In an embodiment, a softmax layer may be defined as:

$$y_j = \frac{e^{x \cdot w_j}}{\sum_{i=0}^{K} e^{x \cdot w_i}}$$

where $w_i \cdot x$ is the output of a neuron just before the non-linearity. A softmax may thus be treated as 1) exponentiate the output of each neuron and 2) normalize the result across the whole layer to obtain probabilities. For two classes, the softmax reduces to the sigmoid function:

$$y_1 = \frac{e^{w_1 \cdot x}}{e^{w_1 \cdot x} + e^{w_2 \cdot x}} = \frac{1}{1 + e^{(w_2 - w_1) \cdot x}}$$

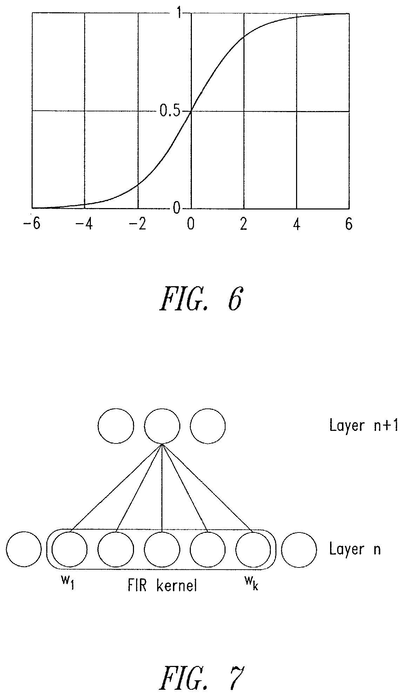

Thus the softmax may be treated as a generalization of the sigmoid / logistic function to multiple dimensions. Figure 6 illustrates an example logistic sigmoid function.

The number of weights required to process an image or a window of a sequential signal grow as $n^2$ where n is the number of input values and thus may lead to overfitting. A convolutional layer may be employed to reduce the number of parameters, and facilitate avoiding overfitting. A convolutional layer may reduce the number of parameters through weight sharing and local connectivity. For example, in weight sharing neurons in layer *n+1* may be connected to the neurons in layer n using the same set of weights. When local connectivity is employed, only the neurons in layer n near the location of the neuron in later n+1 are connected.

Figure 7 illustrates an example of a convolutional layer employing a sliding window. Computing the weighted sum of the previous layer over a sliding window is equivalent to a FIR filtering operation on the previous layer; the layer is thus called convolutional. In the most general case, the layers *n* and n+1 have a number of channels, that is, values referred to the same spatial position (such as stereo channels in audio signals or color channels in images). The convolution is thus extended to all the input channels by applying a different filter to each channel and summing the results. Moreover, a number of output channels are generated by applying a different set of filters to the input channels.

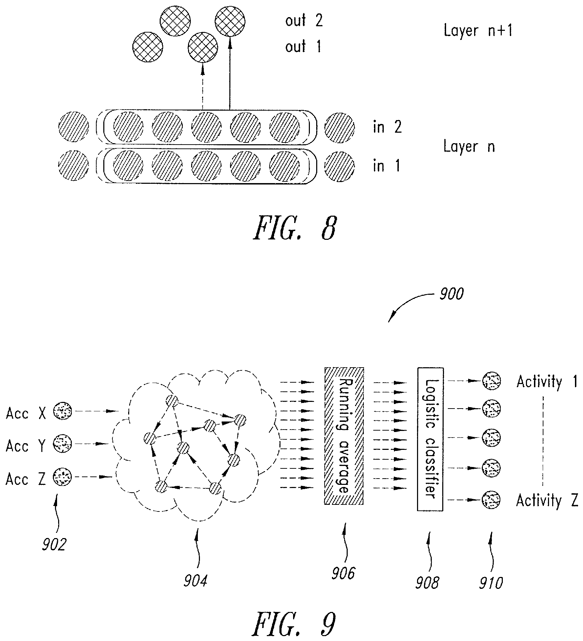

Figure 8 shows the structure of an example convolutional network in a typical case. The filter weights are all learned, so in the design phase only three parameters are specified: the number of input channels c, the number of output channels m and the size of the filter kernel k.

A pooling layer may be employed to down-sample the input in order to introduce shift-invariance (insensitivity to small changes in alignment in the input signal) and to reduce the number of neurons in the subsequent layers. A pooling layer is often used after a convolutional layer and before a fully connected layer. The pooling layer is characterized by the window size N and a pooling function. Typical choices for the pooling function are the *max* function:

$$y = \max_{i=0, \ .. \ N-1} x_i$$

and the average function:

$$y = \frac{1}{N} \sum_{i=0}^{N-1} x_i$$

where x is the output of the previous layer restricted to the pooling window.

An Echo State Network (ESN) may be employed in an embodiment as an alternative to the convolutional neural network. The ESN reservoir is characterized by a hidden state $h_t$ of size $n_r$ and a weight matrix $W_r$ of size $n_r \times n_r$. The network receives an input $\alpha_t$ of size $n_i$ weighted by a matrix $W_{in}$ of size $n_r \times n_i$. Optionally, a bias $b_r$ can be added to each neuron. The matrix $W_r$ may be computed by sampling the coefficients randomly from a uniform distribution in -1, 1: $w_{ij} \sim u([-1,1])$ and randomly setting weights to zero with probability p, where p the sparsity of the weight matrix. For classification tasks, instead of directly classifying the hidden state, an average may be employed. To save memory, the exponential average $\bar{y}_t = (1 - \alpha)y_t + \alpha y_{t-1}$ may be used instead, where $\alpha$ is an averaging factor. The effective window size for averaging may be $1/\alpha$. The hidden state may be determined according to:

$$y_t = [a_t \ h_t] \text{ where } h_t = \sigma(W_{in}a_{t-1} + W_r h_{t-1} + b_r),$$

where $\sigma$ is a nonlinearity (default may be the *sigmoid* function). The output class may be chosen as the maximum value after multiplying the hidden state by the output matrix $W_o$ and adding the bias $b_o$:

$$c = \max_i (W_0 \bar{y}_t + b_0).$$

Figure 9 illustrates an example embodiment of an ESN pipeline 900. This embodiment is not covered by the claims.

For convenience, Figures 9 to 16 are described with reference to acceleration data. Various types of activity-related data may be employed in embodiments, such as acceleration data, orientation data, geographical position data, physiological data, etc., and various combinations thereof. Accelerometer data is received at 902 and provided to the reservoir network at 904. A running average is determined at 906, and logistic classification occurs at 908. The prediction is output at 910.

In neural network architectures the computations are dominated by scalar products, which combine ADD and MUL operations in a predictable pattern. The number of operations is discussed in terms of MADD for simplicity. The number of MUL is equal to the number of MADD, while the number of ADD is slightly lower. For each sample the hidden state is computed, requiring $n_r \cdot (n_r + n_{in} + 1)$ MADDs and $n_r$ nonlinear function evaluations and the exponential average computed with $2 \cdot (n_{in} + n_r)$ MADDs. For classification, $n_o \cdot (n_r + n_{in} + 1)$ MADDs and $n_o$ CMPs.

The weight matrices and biases need to be stored, in an embodiment, $(n_r + n_o) \cdot (n_r + n_{in} + 1)$ floats may be employed. Moreover, buffers for the hidden state, the running average and the classifier output, totaling $n_o + 2 \cdot n_r + n_i$ floats may be employed.

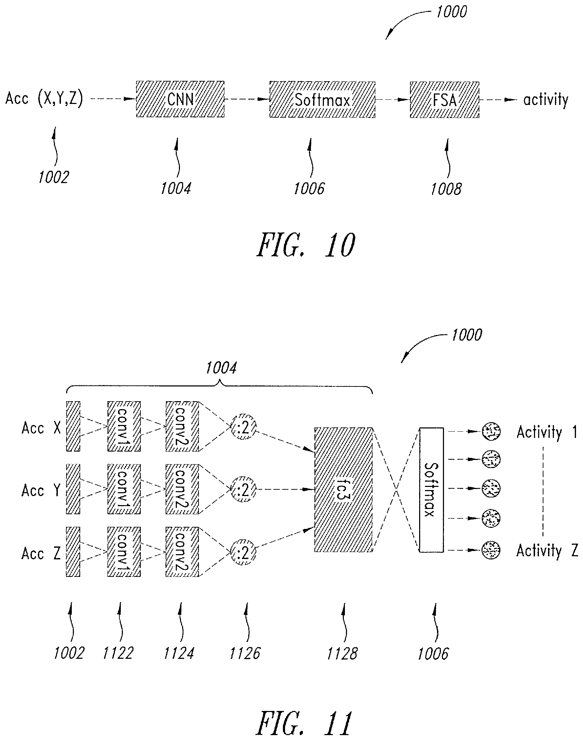

In the study, a convolutional neural network was employed. This model is feed-forward, that is, the output depends only on the input window and not on the temporal history of observations (in contrast with the ESN model). The CNN network in the study was built from m convolutional and n fully connected layers. The first m layers were applied to each acceleration axis separately; the resulting features were max pooled with size and stride of q and then fed to a Multi-Layer Perceptron (MLP) with p hidden neurons (fully connected to the MLP inputs) and r output neurons (one per class). The output of the CNN network was provided to a softmax layer (as a non-limiting example) giving the probability of each class and the class with the highest probability was selected as the prediction. To stabilize the prediction, a state filter (in this case, a finite state automata (FSA)) may be applied. Figure 10 outlines an embodiment of a CNN network pipeline 1000, and Figure 11 illustrates the embodiment 1000 of Figure 10 in more detail. As illustrated in Figure 10, Accelerometer data 1002 is provided to the CNN 1004, the output of the CNN is processed by a softmax layer 1006, and as illustrated, the prediction by the softmax layer 1006 is filtered by an optional FSA filter 1008.

Figure 11 illustrates the CNN and Softmax portions of an embodiment of the CNN 1000 of Figure 10. The convolutional layers 1122, 1124 learn filters of size *k*. The first layer 1122 generates *m* output features and the second layer 1124 generates again n features in output. The features are collected over a window of w samples and windows are separated by a step of s samples. The output of the convolutional layer 1124 is down-sampled by 2 (for example) in a pooling layer 1126. The MLP layer 1128 receives values in input and computes the hidden states. Then, the softmax layer 1006 computes the output as a *softmax* function with z output values, e.g., standing, walking, jogging, biking, driving, etc.

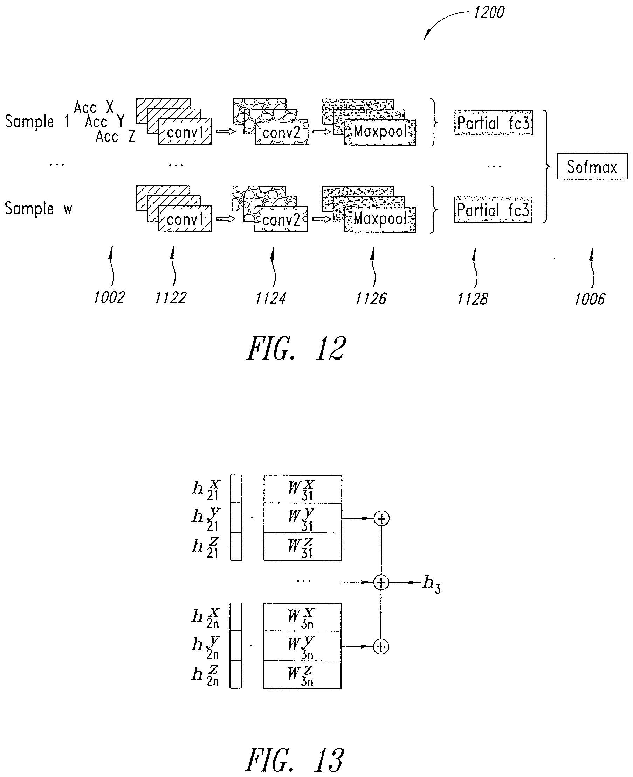

Figure 12 illustrates an embodiment of a partial CNN computation method 1200 that may be employed by the embodiment 1000 of a CNN of Figure 10. While the CNN model uses all of the samples from a window in order to make a decision, the samples can be processed in the convolutional layers as *they arrive* in order to interleave the computation with sensor reading (e.g., when implemented on an embedded platform) and reuse results between overlapping windows. Moreover, the output of the pooling 1126 and the fully-connected layer 3 *(fc3)* 1128 layers can be partially computed as soon as the filtered outputs of the convolutional layers are available for the current sample.

In the study, the three acceleration axes were processed separately through the two convolutional and the pooling layers, producing in output v values per sample. The values were multiplied by a slice $W_{3i}$ of the weight matrix $W_3$ for the fc3 layer and accumulated on the fc3 output $h_3$ until all the samples 1,.., w of the current window were processed. The input values and the weights were re-ordered to match the order of the incoming values and to preserve the results of the matrix multiplication. Figure 13 shows a logical diagram of the operations involved in the partial fc3 computation embodiment of the study.

In the study, the decision was computed by a softmax layer taking $h_3$ as input after all the samples in the window are processed. A single buffer may be employed as long as the step between windows is larger than $4 \cdot (k - 1)$ where k is the size of the convolutional kernel; this condition ensures that the output of each window has no overlap. For shorter steps, more than one buffer and more than one fc3 output would be computed per sample, increasing memory and computational requirements.

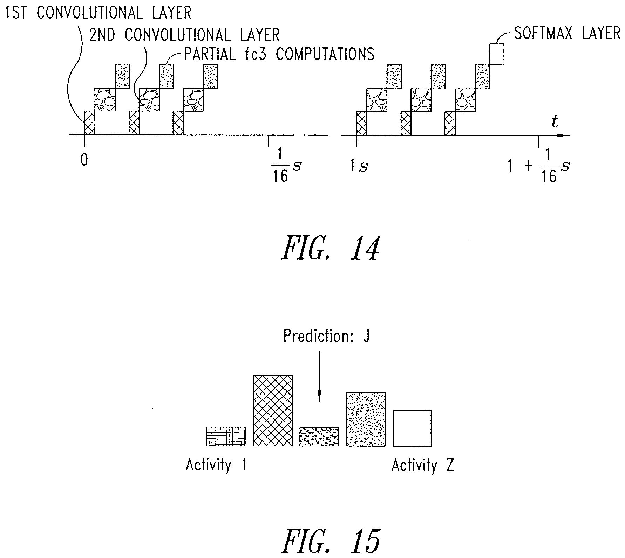

Figure 14 shows how the computational load is distributed, in the scenario where samples are read at a frequency of 16 Hz, and the partial computation of layers *conv1* to fc3 (including an optional *softmax)* can be completed in less than 1/16th of a second.

The CNN output can be affected by transient outliers, that is, predictions that for 1 or two windows give a different value from all the previous predictions because of, e.g., false positives. The outliers can be removed by examining the predictions in a window. This increases accuracy, at the cost of also increasing the latency in the prediction after an activity change. The outliers don't affect significantly the distribution of the predictions over a window, so the choice of an outlier removal mechanism may typically be a median filter with window size *W.*

The use of a median filter, however, may present drawbacks. For example, all the state transitions are treated democratically by a median filter, even if some of the changes (e.g., jogging to driving) are implausible. In addition, an ordering in the activities given by the label codes is assumed, even if that order in meaningless, thus a prediction may be selected even if the prediction is underrepresented, because the prediction is in the middle of the distribution. Figure 15 illustrates an example histogram of a label distribution on a window and a label selected by a median filter. In Figure 15, the prediction is Activity 3, which is underrepresented in the window, but which is in the center of the window.

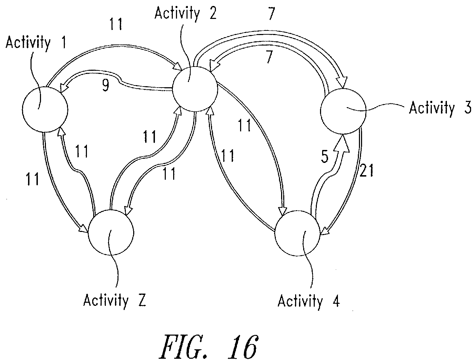

To facilitate addressing the drawbacks of a median filter, the likelihood of the transitions can be encoded in a variable window size, with longer windows selected for unlikely transitions. The transitions may be encoded in a Finite State Machine (FSM), also referred to as a Finite State Automata (FSA), where the states represent a confirmed prediction and the edges encode the window size to confirm a transition to another state. An example embodiment of an FSM (or FSA) is illustrated in Figure 16. The dependence on the label order may be removed by using a majority filter, where a label is selected if at least 50% of the predictions in the window agree on a specific outcome.

The FSM (or FSA) may be implemented as a transition matrix *T,* where the element $t_{ij}$ represents the window size from node *i* to node *j* and a special value *(e.g.,* infinity) indicates that no transition is allowed. For transitions into the same state, a conventional value of 0 may be used. A queue of the last $t_{max}$ predictions may be maintained, where $t_{max}$ the maximum window size, and a transition from *i* to *j* is allowed if in the last $t_{ij}$ predictions, at least $\frac{t_{ij}}{2} + 1$ *(consensus threshold)* agree on the *j* label. If $t_{ij}$ is the special value or no transition reaches the consensus threshold, the state is not changed.

It is noted that the neural network models may be trained end-to-end and do not require any specific parameter tuning, development of ad hoc rules, features, classification steps, etc. This may be advantageous in some embodiments. For example neural network embodiment may employ shorter design times for a classifier assuming a growing number of activities to be recognized from time to time. In addition, training tools optimized for powerful processor designs may significantly shorten the training times.

The Dataset 2.0 has a skewed class distribution, where the stationary class is overrepresented. As the stationary activity is much more likely than all the other activities, it makes sense to use a larger set of examples and adopt a training strategy that minimizes false negatives for the stationary class. For neural networks the same mechanism may be implemented explicitly (e.g. hard negative mining).

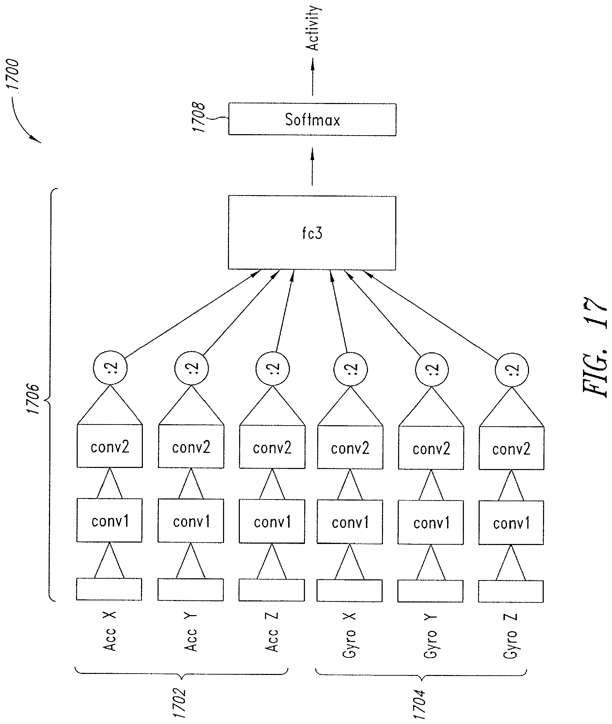

As mentioned above, the wearable device 110 of Figure 1 may comprise one or more other sensors, such as one or more gyroscopes 160, in addition to one or more accelerometers 122. Figure 17 illustrates CNN and Softmax portions of an embodiment of a CNN 1700, similar to the embodiment 1000 of Figures 10 and 11, but which processes accelerometer and gyroscope data. As illustrated in Figure 17, Accelerometer data 1702 and gyroscope data 1704 are provided to the CNN 1706, the output of the CNN is processed by a softmax layer 1708. The prediction by the softmax layer 1708 may be filtered by an optional FSA filter (see FSA filter 1008 of Figure 10). The training data set including acceleration and orientation data used to train the activity-classification model may be equalized with respect to the classifications. In some embodiments, additional activity-related data may be considered in the classification model, such as data from one or more physiological sensors, such as the physiological sensor 124 of Figure 1, data from one or more position sensors, such as the position sensor 126 of Figure 1, etc., and various combinations thereof.

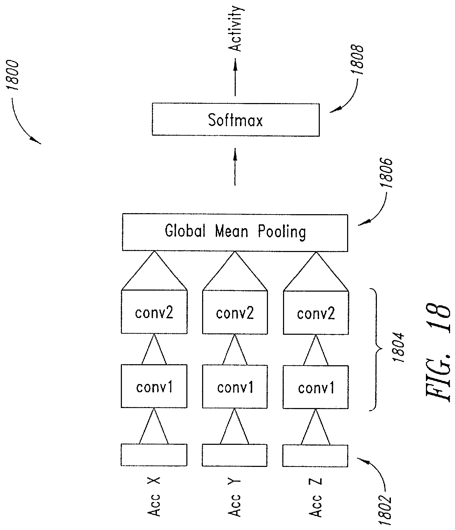

Figure 18 illustrates CNN and Softmax portions of an embodiment of a CNN 1800, similar to the embodiment 1000 of Figures 10 and 11, where the fc3 layer has been replaced with a global mean pooling layer 1806. As illustrated in Figure 18, Accelerometer data 1802 are provided to a series of convolutional layer 1804, an output of the series of convolutional layers 1804 is provided to the global mean pooling layer 1806. The global mean pooling layer selects the mean values across all the convolutional channels and the current window and provides these values to the softmax layer 1808. The prediction by the softmax layer 1808 may be filtered by an optional FSA filter (see FSA filter 1008 of Figure 10). Some embodiments may employ other types of global pooling layers, such as a global maximum pooling layer, a global minimum pooling layer, etc. Using a global pooling layer instead of an fc3 layer may facilitate significantly reducing the number of parameters and operations. In some embodiments, using a global mean pooling layer instead of an fc3 layer may reduce the number of parameters by a factor of 10, and the number of operations by a factor of 2.

Some embodiments may take the form of or include computer program products. For example, according to one embodiment there is provided a computer readable medium including a computer program adapted to perform one or more of the methods or functions described above. The medium may be a physical storage medium such as for example a Read Only Memory (ROM) chip, or a disk such as a Digital Versatile Disk (DVD-ROM), Compact Disk (CD-ROM), a hard disk, a memory, a network, or a portable media article to be read by an appropriate drive or via an appropriate connection, including as encoded in one or more barcodes or other related codes stored on one or more such computer-readable mediums and being readable by an appropriate reader device.

Furthermore, in some embodiments, some of the systems and/or modules and/or circuits and/or blocks may be implemented or provided in other manners, such as at least partially in firmware and/or hardware, including, but not limited to, one or more application-specific integrated circuits (ASICs), digital signal processors, discrete circuitry, logic gates, standard integrated circuits, state machines, look-up tables, controllers (e.g., by executing appropriate instructions, and including microcontrollers and/or embedded controllers), field-programmable gate arrays (FPGAs), complex programmable logic devices (CPLDs), etc., as well as devices that employ RFID technology, and various combinations thereof.

The various embodiments described above can be combined to provide further embodiments. Aspects of the embodiments can be modified, if necessary to employ concepts of the various patents, applications and publications to provide yet further embodiments.

These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

**Claims**

1. A method 2. implemented on a device for recognizing human activity based on sensed information, comprising:

   receiving (112, 114) human activity-related data sequences of samples (122 124, 126, 160);
   determining ( 158) activity classifications on the received activity-related data sequences based on an activity-classification model trained using a classification-equalized training data set (212); and
   generating a classification signal (300) based on the determined classifications,
   wherein the classification-equalized training data set (212) comprises a first class having a plurality of human activity-related data sequences of a first sequence length and a total number of samples N, and one or more additional classes having a plurality of human activity-related data sequences, each class having a respective truncated sequence length $t_j$ and a total respective number of samples $N_j$ , wherein the sequence truncation satisfies:

   $N_j \geq N$, for sequence length $t_j$; and
   $N_j < N$, for sequence length $t_j$-1,
   wherein the method further comprises generating (200; 202 to 212) the classification-equalized training data set by:

   - determining (204) a class $c_i$ in a training dataset having a fewest number of samples N
   - determining (206) the number of samples N in the determined class $c_i$;
   - determining (208), for each class $c_j$ in the training dataset other than the determined class

   $c_i$, a sequence length $t_j$ such that the total number of samples $N_j$ for the class
   $c_j$, assuming the sequences of the class $c_j$ are truncated at sequence length
   $t_j$, equals or just exceeds the number N of samples in the class $c_i$;

   - for each class $c_j$ other than the determined class $c_i$, truncating (210) all of the sequences in class $c_j$ to the determined sequence length $t_j$ for the class $c_j$, wherein samples in each sequence for each class $c_j$ beyond the determined length $t_j$ are discarded;
   - returning (212) the classification-equalized training data set,

   and wherein the method further comprises training the activity classification model (156) using the classification-equalized training dataset
   and wherein both determining 158) activity classifications and training (156) activity-classification model using the classification-equalized training data set (212) comprise using a convolutional neural network using as input a window of human activity-related data sequence, said window having a predetermined number of samples.

2. The method of claim 1 wherein the determining activity classifications comprises extracting feature data (302) based on received accelerometer data.

3. The method of claim 1 wherein the convolutional neural network (152; 1000) has a feed-forward architecture.

4. The method of claim 3 wherein the convolutional neural network (1000) comprises a layered architecture including one or more of:

   one or more convolutional layers (1122, 1124);
   one or more pooling layers (1126); and
   one or more softmax layers (1006).

5. The method of any of claims 3 or 4 wherein the convolutional neural network (152) comprises a finite state machine.

6. The method of claim 1 wherein the activity-related data comprises one or more of:

   acceleration data (122);
   orientation data (160);
   geographical position data (126); and
   physiological data (124).

7. A device, comprising:

   an interface (112, 114), which, in operation, receives one or more signals indicative of activity (122, 124, 126, 160); and
   signal processing circuitry (150, 190), which, in operation:

      determines (152, 158) activity classifications based on the received signals indicative of activity and an activity-classification model trained using a classification-equalized training data set (212); and
      generates a classification signal (300) based on the determined classifications,
      wherein the device is configured for operating with the method of any of claims 1 to 6.

8. A system, comprising:

   one or more sensors (122, 124, 126, 160), which, in operation, generate one or more activity-related signals; and
   the device of claim 7 with said interface (112, 114), coupled with the one or more sensors to receive therefrom said one or more signals indicative of activity.

9. The system of claim 8
   wherein the one or more sensors (122, 124, 126, 160) comprise a memory and one or more processor cores, wherein the memory stores contents which in operation configure the one or more processor cores to generate the activity classification signal.

**Patentansprüche**

1. Verfahren, das auf einer Vorrichtung implementiert wird, um auf Basis von erfassten Informationen menschliche Aktivität zu erkennen, umfassend:

   Empfangen (112, 114) von mit menschlicher Aktivität zusammenhängenden Datensequenzen aus Abtastungen (122, 124, 126, 160);
   Bestimmen (158) von Aktivitätsklassifikationen an den empfangenen, mit Aktivität zusammenhängenden Datensequenzen auf Basis eines Aktivitätsklassifikationsmodells, das unter Verwendung eines klassifikationsausgeglichenen Trainingsdatensatzes (212) trainiert wurde; und

Erzeugen eines Klassifikationssignals (300) auf Basis der bestimmten Klassifikationen,
wobei der klassifikationsausgeglichene Trainingsdatensatz (212) eine erste Klasse mit einer Vielzahl von mit menschlicher Aktivität zusammenhängenden Datensequenzen einer ersten Sequenzlänge und einer Gesamtanzahl von Abtastungen N, und eine oder mehrere zusätzliche Klassen mit einer Vielzahl von mit menschlicher Aktivität zusammenhängenden Datensequenzen umfasst, wobei jede Klasse eine jeweilige gekürzte Sequenzlänge $t_j$ und eine jeweilige Gesamtanzahl von Abtastungen $N_j$ aufweist, wobei die Sequenzkürzung erfüllt:

$N_j \geq N$ bei Sequenzlänge $t_j$; und
$N_j < N$ bei Sequenzlänge $t_j$-1,
wobei das Verfahren weiter umfasst
Erzeugen (200; 202 bis 212) des klassifikationsausgeglichenen Trainingsdatensatzes durch:

- Bestimmen (204) einer Klasse $c_i$ in einem Trainingsdatensatz mit einer kleinsten Anzahl von Abtastungen N;
- Bestimmen (206) der Anzahl von Abtastungen N in der bestimmten Klasse $c_i$;
- Bestimmen (208), für jede Klasse $c_j$ in dem Trainingsdatensatz, bei der es sich nicht um die bestimmte Klasse $c_i$ handelt, einer Sequenzlänge $t_j$ derart, dass die Gesamtanzahl von Abtastungen $N_j$ bei der Klasse $c_j$, unter der Annahme, dass die Sequenzen der Klasse $c_j$ auf Sequenzlänge $t_j$ gekürzt sind, gleich der Anzahl N von Abtastungen in der Klasse $c_i$ ist oder diese gerade übersteigt;
- Kürzen (210), bei jeder Klasse $c_j$, bei der es sich nicht um die bestimmte Klasse $c_i$ handelt, aller Sequenzen in Klasse $c_j$ auf die bestimmte Sequenzlänge $t_j$ für die Klasse $c_j$, wobei Abtastungen in jeder Sequenz bei jeder Klasse $c_j$, die über die bestimmte Länge $t_j$ hinausgehen, verworfen werden;
- Zurückgeben (212) des klassifikationsausgeglichenen Trainingsdatensatzes,

und wobei das Verfahren weiter das Trainieren des Aktivitätsklassifikationsmodells (156) unter Verwendung des klassifikationsausgeglichenen Trainingsdatensatzes umfasst,
und wobei sowohl das Bestimmen (158) von Aktivitätsklassifikationen als auch Trainieren (156) des Aktivitätsklassifikationsmodells unter Verwendung des klassifikationsausgeglichenen Trainingsdatensatzes (212) das Verwenden eines neuronalen Faltungsnetzwerks umfasst,
das als Eingabe ein Fenster einer mit menschlicher Aktivität zusammenhängenden Datensequenz verwendet, wobei das Fenster eine vorbestimmte Anzahl von Abtastungen aufweist.

2. Verfahren nach Anspruch 1, wobei das Bestimmen von Aktivitätsklassifikationen das Extrahieren von Merkmalsdaten (302) auf Basis von empfangenen Beschleunigungsmesserdaten umfasst.

3. Verfahren nach Anspruch 1, wobei das neuronale Faltungsnetzwerk (152; 1000) eine vorwärtsgerichtete Architektur aufweist.

4. Verfahren nach Anspruch 3, wobei das neuronale Faltungsnetzwerk (1000) eine geschichtete Architektur umfasst, die eines oder mehrere einschließt aus:

einer oder mehreren Faltungsschichten (1122, 1124);
einer oder mehreren Poolingschichten (1126); und
einer oder mehreren Softmax-Schichten (1006).

5. Verfahren nach einem der Ansprüche 3 oder 4, wobei das neuronale Faltungsnetzwerk (152) einen endlichen Automaten umfasst.

6. Verfahren nach Anspruch 1, wobei die mit Aktivität zusammenhängenden Daten eines oder mehrere umfassen aus:

Beschleunigungsdaten (122);
Ausrichtungsdaten (160);
geographischen Positionsdaten (126); und
physiologischen Daten (124).

7. Vorrichtung, umfassend:

eine Schnittstelle (112, 114), die im Betrieb ein oder mehrere Signale empfängt, die Aktivität (122, 124, 126, 160) anzeigen; und
Signalverarbeitungsschaltungen (150, 190), die im Betrieb:

auf Basis der empfangenen, Aktivität anzeigenden Signale und eines unter Verwendung eines klassifikationsausgeglichenen Trainingsdatensatzes (212) trainierten Aktivitätsklassifikationsmodells Aktivitätsklassifikationen bestimmen (152, 158); und
auf Basis der bestimmten Klassifikationen ein Klassifikationssignal (300) erzeugen,
wobei die Vorrichtung so konfiguriert ist, dass sie mit dem Verfahren nach einem der Ansprüche 1 bis 6 arbeitet.

8. System, umfassend:

einen oder mehrere Sensoren (122, 124, 126, 160), die im Betrieb ein oder mehrere mit Aktivität zusammenhängende Signale erzeugen; und
die Vorrichtung nach Anspruch 7 mit der Schnittstelle (112, 114), die mit dem einen oder den mehreren Sensoren gekoppelt ist, um von diesen das eine oder die mehreren, Aktivität anzeigenden Signale zu empfangen.

9. System nach Anspruch 8, wobei der eine oder die mehreren Sensoren (122, 124, 126, 160) einen Speicher und einen oder mehrere Prozessorkerne umfassen, wobei der Speicher Inhalte speichert, die im Betrieb den einen oder die mehreren Prozessorkerne so konfigurieren, dass sie das Aktivitätsklassifikationssignal erzeugen.

## Revendications

1. Procédé mis en œuvre sur un dispositif pour la reconnaissance d'activité humaine sur la base d'informations détectées, comprenant :

la réception (112, 114) de séquences de données relatives à l'activité humaine constituées d'échantillons (122, 124, 126, 160) ;
la détermination (158) de classifications d'activité sur les séquences de données relatives à l'activité reçues sur la base d'un modèle de classification d'activité entraîné à l'aide d'un ensemble de données d'entraînement égalisé quant aux classifications (212) ; et
la génération d'un signal de classification (300) sur la base des classifications déterminées,
dans lequel l'ensemble de données d'entraînement égalisé quant aux classifications (212) comprend une première classe ayant une pluralité de séquences de données relatives à l'activité humaine d'une première longueur de séquence et un nombre total d'échantillons N, et une ou plusieurs classes supplémentaires ayant une pluralité de séquences de données relatives à l'activité humaine, chaque classe ayant une longueur de séquence tronquée respective $t_j$ et un nombre total d'échantillons respectif $N_j$, la troncature de séquence satisfaisant :

$N_j \geq N$, pour une longueur de séquence $t_j$ ; et
$N_j < N$, pour une longueur de séquence $t_j - 1$,
le procédé comprenant en outre la génération (200 ; 202 à 212) de l'ensemble de données d'entraînement égalisé quant aux classifications par :

- détermination (204) d'une classe c, dans un ensemble de données d'entraînement ayant un nombre d'échantillons N le plus petit ;
- détermination (206) du nombre d'échantillons $N$ dans la classe déterminée c, ;
- détermination (208), pour chaque classe $c_j$ dans l'ensemble de données d'entraînement autre que la classe déterminée $c_i$, d'une longueur de séquence $t_j$ telle que le nombre total d'échantillons $N_j$ pour la classe $c_j$, en supposant que les séquences de la classe $c_j$ sont tronquées à une longueur de séquence $t_j$, soit égal au nombre N d'échantillons dans la classe c, ou le dépasse juste ;
- pour chaque classe $c_j$ autre que la classe déterminée $c_i$, troncature (210) de toutes les séquences dans la classe $c_j$ à la longueur de séquence déterminée $t_j$ pour la classe $c_j$, des échantillons dans chaque séquence pour chaque classe $c_j$ au-delà de la longueur déterminée $t_j$ étant abandonnés ;
- renvoi (212) de l'ensemble de données d'entraînement égalisé quant aux classifications,

et le procédé comprenant en outre l'entraînement du modèle de classification d'activité (156) à l'aide de l'ensemble de données d'entraînement égalisé quant aux classifications, dans lequel la détermination (158) de classifications d'activité et l'entraînement (156) du modèle de classification d'activité à l'aide de l'ensemble de données d'entraînement égalisé quant aux classifications (212) comprennent tous les deux l'utilisation d'un réseau de neurones convolutif utilisant comme entrée une fenêtre de séquence de données relatives à l'activité humaine, ladite fenêtre ayant un nombre d'échantillons prédéterminé.

2. Procédé selon la revendication 1, dans lequel la détermination de classifications d'activité comprend l'extraction de données de caractéristiques (302) sur la base de données d'accéléromètre reçues.

3. Procédé selon la revendication 1, dans lequel le réseau de neurones convolutif (152 ; 1000) a une architecture à propagation avant.

4. Procédé selon la revendication 3, dans lequel le réseau de neurones convolutif (1000) comprend une architecture en couches comportant un ou plusieurs éléments parmi :

une ou plusieurs couches de convolution (1122, 1124) ;
une ou plusieurs couches de regroupement (1126) ; et
une ou plusieurs couches softmax (1006).

5. Procédé selon l'une quelconque des revendications 3 et 4, dans lequel le réseau de neurones convolutif (152) comprend un automate fini.

6. Procédé selon la revendication 1, dans lequel les données relatives à l'activité comprennent un ou plusieurs éléments parmi :

des données d'accélération (122) ;
des données d'orientation (160) ;
des données de position géographique (126) ; et
des données physiologiques (124).

7. Dispositif, comprenant :

une interface (112, 114) qui, en fonctionnement, reçoit un ou plusieurs signaux indicatifs d'activité (122, 124, 126, 160) ; et
une circuiterie de traitement de signaux (150, 190) qui, en fonctionnement :

détermine (152, 158) des classifications d'activité sur la base des signaux indicatifs d'activité reçus et d'un modèle de classification d'activité entraîné à l'aide d'un ensemble de données d'entraînement égalisé quant aux classifications (212) ; et
génère un signal de classification (300) sur la base des classifications déterminées,
le dispositif étant configuré pour fonctionner avec le procédé selon l'une quelconque des revendications 1 à 6.

8. Système, comprenant :

un ou plusieurs capteurs (122, 124, 126, 160) qui, en fonctionnement, génèrent un ou plusieurs signaux relatifs à l'activité ; et
le dispositif selon la revendication 7 doté de ladite interface (112, 114), couplé aux un ou plusieurs capteurs pour en recevoir lesdits un ou plusieurs signaux indicatifs d'activité.

9. Système selon la revendication 8, dans lequel les un ou plusieurs capteurs (122, 124, 126, 160) comprennent une mémoire et un ou plusieurs cœurs de processeur, la mémoire stockant des contenus qui, en fonctionnement, configurent les un ou plusieurs cœurs de processeurs pour générer le signal de classification d'activité.

FIG. 1

_— 200_

_— 202_

DETERMINE CLASS $c_i$ WITH LEAST NUMBER OF SAMPLES | _— 204_

DETERMINE NUMBER OF SAMPLES N IN CLASS $c_i$ | _— 206_

FOR EACH CLASS $c_j$, DETERMINE SEQUENCE LENGTH $t_j$ | _— 208_

TRUNCATE EACH CLASS $c_j$ TO DETERMINED SEQUENCE LENGTH $t_j$ | _— 210_

_— 212_

*FIG. 2*

FIG. 3

EP 3 301 611 B1

FIG. 4

FIG. 5

FIG. 6

Layer n+1

Layer n

$w_1$    FIR kernel    $w_k$

FIG. 7

out 2

out 1

Layer n+1

in 2

in 1

Layer n

## FIG. 8

900

Acc X

Acc Y

Acc Z

Running average

Logistic classifier

Activity 1

Activity Z

902

904

906

908

910

## FIG. 9

Acc (X,Y,Z) - - - ▶ CNN - - -▶ Softmax -▶ FSA - -▶ activity

1002    1004    1006    1008

*FIG. 10*

1004

Acc X    conv1    conv2    :2

Acc Y    conv1    conv2    :2    fc3    Softmax    Activity 1

Acc Z    conv1    conv2    :2    Activity Z

1002  1122  1124  1126    1128    1006

*FIG. 11*

**FIG. 12**

**FIG. 13**

1ST CONVOLUTIONAL LAYER
2ND CONVOLUTIONAL LAYER
PARTIAL fc3 COMPUTATIONS
SOFTMAX LAYER

$t$

$0$    $\dfrac{1}{16}s$    $1s$    $1 + \dfrac{1}{16}s$

*FIG. 14*

Prediction: J

Activity 1    Activity Z

*FIG. 15*

24

FIG. 16

*FIG. 17*

*FIG. 18*

**REFERENCES CITED IN THE DESCRIPTION**

**Non-patent literature cited in the description**

- **MUHAMMAD SHAOIB et al.** Towards Physical Activity Recognition Using Smartphone Sensors. *2013 IEEE 10th International Conference on Ubiquitous Intelligence and Computing, UIC,* 2013, 80-87
- **FRANCISCO ORDÓÑEZ et al.** Deep Convolutional and LSTM recurrent Neural Networks for Multimodal Wearable Activity recognition. *Sensors,* vol. 16 (1), 115
- **ANDREAS BULLIG et al.** A tutorial on human activity recognition using body-worn inertial sensor. *ACM Computing Surveys, ACM, New York,* vol. 46 (3), 1-33